# EUROPEAN PATENT APPLICATION

(11) **EP 4 403 160 A1**
(43) Date of publication of application: **24.07.2024**
(21) Application number: 22870059.7
(22) Date of filing: 16.09.2022
(51) Int. Cl.: A61K 6/60, A61C 7/08, A61C 13/01, A61C 13/07, A61C 13/15

(54) **METHOD FOR MANUFACTURING INTRAORAL APPLIANCE EQUIPPED WITH COATING, AND INTRAORAL APPLIANCE EQUIPPED WITH COATING**

(30) Priority: 17.09.2021 JP 2021152629
(71) Applicant: Kuraray Noritake Dental Inc., Kurashiki-shi, Okayama 710-0801 (JP)
(72) Inventor: SUZUKI, Kenji, Tainai-shi, Niigata 959-2653 (JP)
(74) Representative: D Young & Co LLP
(86) International application number: PCT/JP2022/034807
(87) International publication number: WO 2023/042912

(57) **Abstract**

The present invention provides a method of producing an intraoral appliance equipped with a coated film including an intraoral appliance that is a stereolithographic article of a stereolithographic curable composition having formed on a prescribed surface thereof a coated film capable of imparting excellent adhesion durability and toughness, and relates to a method of producing an intraoral appliance equipped with a coated film, including coating on a prescribed surface of an intraoral appliance that is a stereolithographic article of a stereolithographic curable composition (A) containing a (meth)acrylic based polymerizable monomer (a) and a photopolymerization initiator (b), a curable composition (B) containing the same (meth)acrylic based polymerizable monomer (a) as contained in the stereolithographic curable composition (A), so as to form a coated film of a resin (B 1) containing a polymer having a structure derived from the same (meth)acrylic based polymerizable monomer (a) as contained in the stereolithographic curable composition (A), on the prescribed surface of the intraoral appliance.

## Description

### Technical Field

The present invention relates to a method of producing an intraoral appliance equipped with a coated film including an intraoral appliance that is a stereolithographic article having formed on a prescribed surface thereof a coated film, and an intraoral appliance equipped with a coated film.

### Background Art

An intraoral appliance means a therapeutic device that is used by inserting into the alveolar ridge or dentition, and examples thereof mainly include a denture base, a dental occlusal splint, and a mouthpiece-shaped sleep disorder treatment equipment. Among these, a denture base is being widely used for particularly a large number of patients. A denture is obtained by mounting a prosthetic tooth on a denture base. The denture is also encompassed in the intraoral appliance.

In the case where a denture is worn for a long period of time, the surface lubricity of the denture base is decreased due to the influence of contamination and abrasion from foods and drinks, contamination from oral bacteria, and brushing and detergent in cleaning. Therefore, the denture base is re-polished, or a coated film (which may also be referred to as a coating) is re-formed on the surface of the denture base for the purposes of glazing, anti-fouling, and anti-bacterial, but since the polishing requires skill and is labor intensive, a coating, which can be easily formed, is used in some cases. Furthermore, the denture being worn becomes unadapted to the oral mucosa due to bone resorption, deformation of the mucosal surface, and the like. Accordingly, a means of re-fitting the denture by coating a backing material on the alveolar ridge-contact surface of the denture base having been unadapted is being clinically used.

Examples of the material used in the denture base include an acrylic based resin, a polycarbonate based resin, and a polysulfonic acid based resin, and an acrylic based resin containing polymethyl methacrylate or polyethyl methacrylate as a main material is being most frequently used. The material that is used for the coating is generally a (meth)acrylic based monomer, and most of the material that is used for the backing material is a mixture of (meth)acrylic based monomer and polymer, and it is no exaggeration to say that the mixture is substantially exclusively therefor.

The mainstream of the ordinary production method of a denture base is a method of using a mixture of methyl methacrylate (MMA) and polymethyl methacrylate (PMMA) as a denture base material, and polymerizing the mixture under heat to provide a denture base. A denture base material can also be produced by stereolithography, which is being rapidly spread according to the progress of the stereolithographic apparatus and the stereolithographic material. The stereolithography necessitates the use of a polymerizable compound having particularly high curability as the denture base material from the standpoint of curing with light instantaneously, and a crosslinkable monomer and a monomer exhibiting high cohesive force are used therefor. A volatile component cannot be used in view of the structure of the stereolithographic apparatus, and therefore the use of MMA is unpractical. For these factors, the denture base that is a stereolithographic article has a high crosslinking density, which prevents the coating material and the adhesive for adhering the backing material from penetrating thereto, resulting in a problem that the coating and the backing are difficult to apply to the denture base that is a stereolithographic article.

Furthermore, an intraoral appliance, such as a denture, is wholly or partially deformed due to the mechanical load, such as occlusion and clenching, which results in a problem that the coated film on the intraoral appliance produced by stereolithography is easily peeled off, or the coated film is cracked even if not peeled off, and the crack is expanded to provide a tendency of breaking the appliance entirely.

The dental adhesive material (adhesive composition) is widely known to encompass a material to be applied to the tooth substance, a material to be applied to a dental composite resin, and also a material to be applied to a metal or ceramic dental prosthesis. The dental adhesive material used for these applications includes a material that has high affinity to the adherends, such as the tooth substance, and forms chemical bond thereto, for example, an acidic monomer, a silane coupling agent, and a sulfur-containing monomer. However, these dental adhesive materials are based on the techniques and the expression mechanism of adhesiveness that are completely different from the adhesive materials to be applied to an intraoral appliance, such as a denture base, a dental occlusal splint, and a sleep disorder treatment equipment. Therefore, it is difficult to divert the former directly to the latter.

In view of the background described above, as a surface treatment method of a denture base not relying on the ordinary methods, PTL 1 describes a composition excellent in adhesiveness containing a mixture of monofunctional and polyfunctional (meth)acrylic based monomers diluted with a solvent, and PTL 2 describes a photocurable backing material excellent in curability. PTL 3 describes a denture base material for stereolithography excellent in strength and toughness. Furthermore, PTLs 4 and 5 each describe a technique of smoothing the surface of a stereolithographic article or making a stereolithographic article transparent, by forming a coated film on the surface of the stereolithographic article with the same resin composition as the stereolithographic article.

### Citation List

### Patent Literatures

PTL 1: JP2008-214359A
PTL 2: JP2019-44065A
PTL 3: WO2018/181832
PTL 4: JPH2-24122 A
PTL 5: JP2000-318048A

### Summary of Invention

### Technical Problem

However, PTLs 1 and 2 target backing of a denture base produced with a PMMA based denture base material, and these literatures fail to describe the use of a stereolithographic material having a high crosslinking system applied to stereolithography, and the backing targeting a denture base formed of a stereolithographic article produced by such a stereolithographic material. Furthermore, PTLs 3 to 5 fail to describe the use of a stereolithographic material as a backing material for a denture base or an adhesive material for backing of a denture base.

Under the circumstances, an object of the present invention is to provide a method of producing an intraoral appliance equipped with a coated film including an intraoral appliance that is a stereolithographic article of a stereolithographic curable composition having formed on a prescribed surface thereof a coated film capable of imparting excellent adhesion durability and toughness. Another object thereof is to provide an intraoral appliance equipped with a coated film including an intraoral appliance that is a stereolithographic article of a stereolithographic curable composition having formed on a prescribed surface thereof a coated film capable of imparting excellent adhesion durability and toughness.

### Solution to Problem

The present invention encompasses the following inventions.
[1] A method of producing an intraoral appliance equipped with a coated film, including coating on a prescribed surface of an intraoral appliance that is a stereolithographic article of a stereolithographic curable composition (A) containing a (meth)acrylic based polymerizable monomer (a) and a photopolymerization initiator (b), a curable composition (B) containing the same (meth)acrylic based polymerizable monomer (a) as contained in the stereolithographic curable composition (A), so as to form a coated film of a resin (B 1) containing a polymer having a structure derived from the same (meth)acrylic based polymerizable monomer (a) as contained in the stereolithographic curable composition (A), on the prescribed surface of the intraoral appliance.
[2] The method of producing an intraoral appliance equipped with a coated film according to the item [1], in which the curable composition (B) contains the same (meth)acrylic based polymerizable monomer (a) as contained in the stereolithographic curable composition (A) in a mass proportion in effective ingredients of 10% by mass or more.
[3] The method of producing an intraoral appliance equipped with a coated film according to the item [1] or [2], in which the method includes coating the curable composition (B) on an alveolar ridge-contact surface, which is a surface to be brought into contact with an alveolar ridge, of the intraoral appliance, so as to form a coated film of the resin (B 1) on the alveolar ridge-contact surface of the intraoral appliance.
[4] The method of producing an intraoral appliance equipped with a coated film according to any one of the items [1] to [3], in which the stereolithographic curable composition (A) and the curable composition (B) contain a polyfunctional (meth)acrylic based polymerizable monomer (a-1) as the (meth)acrylic based polymerizable monomer (a).
[5] The method of producing an intraoral appliance equipped with a coated film according to the item [4], in which the polyfunctional (meth)acrylic based polymerizable monomer (a-1) contains a urethanated (meth)acrylic compound.
[6] The method of producing an intraoral appliance equipped with a coated film according to the item [5], in which the stereolithographic curable composition (A) and the curable composition (B) further contain a monofunctional (meth)acrylic based polymerizable monomer (a-2) as the (meth)acrylic based polymerizable monomer (a).
[7] The method of producing an intraoral appliance equipped with a coated film according to the item [6], in which the monofunctional (meth)acrylic based polymerizable monomer (a-2) contains a (meth)acrylate ester based polymerizable monomer containing an aromatic ring.
[8] The method of producing an intraoral appliance equipped with a coated film according to any one of the items [1] to [7], in which the intraoral appliance has been subjected to secondary polymerization after stereolithography, and the method includes coating the curable composition (B) on a prescribed surface of the stereolithographic article having been subjected to secondary polymerization, so as to form a coated film of the resin (B1) on the prescribed surface.
[9] The method of producing an intraoral appliance equipped with a coated film according to any one of the items [1] to [7], in which the method includes, after stereolithography of the stereolithographic curable composition (A), coating the curable composition (B) on a prescribed surface of the stereolithographic article before secondary polymerization, so as to form a coated film of the resin (B1) on the prescribed surface.
[10] The method of producing an intraoral appliance equipped with a coated film according to any one of the items [1] to [9], in which the method includes machining the coated film after forming the coated film.
[11] An intraoral appliance equipped with a coated film, including
   an intraoral appliance that is a stereolithographic article of a stereolithographic curable composition (A) containing a (meth)acrylic based polymerizable monomer (a) and a photopolymerization initiator (b), and
   a coated film of a resin (B1) containing a polymer having a structure derived from the same (meth)acrylic based polymerizable monomer (a) as contained in the stereolithographic curable composition (A), formed on a prescribed surface of the intraoral appliance.
[12] The intraoral appliance equipped with a coated film according to the item [11], in which the resin (B1) contains the polymer having a structure derived from the same (meth)acrylic based polymerizable monomer (a) as contained in the stereolithographic curable composition (A) in an amount of 50% by mass or more.
[13] The intraoral appliance equipped with a coated film according to the item [11] or [12], in which the intraoral appliance has the coated film of the resin (B1) on an alveolar ridge-contact surface, which is a surface to be brought into contact with an alveolar ridge, of the intraoral appliance.
[14] The intraoral appliance equipped with a coated film according to any one of the items [11] to [13], in which the intraoral appliance is a denture base.
[15] The intraoral appliance equipped with a coated film according to any one of the items [11] to [14], in which the coated film has machining marks.

### Advantageous Effects of Invention

The present invention can provide a method of producing an intraoral appliance equipped with a coated film including an intraoral appliance that is a stereolithographic article of a stereolithographic curable composition having formed on a prescribed surface thereof a coated film capable of imparting excellent adhesion durability and toughness, and can also provide an intraoral appliance equipped with a coated film including an intraoral appliance that is a stereolithographic article of a stereolithographic curable composition having formed on a prescribed surface thereof a coated film capable of imparting excellent adhesion durability and toughness.

### Brief Description of Drawings

Fig. 1 is a plan view of an intraoral appliance equipped with a coated film according to the present embodiment.
Fig. 2 is a schematic cross sectional view of the intraoral appliance equipped with a coated film in Fig. 1.
Fig. 3 is a schematic process chart showing one example of the method of producing an intraoral appliance equipped with a coated film according to the present embodiment.
Fig. 4 is a schematic process chart showing another example of the method of producing an intraoral appliance equipped with a coated film.

### Description of Embodiments

In the description herein, the upper limit values and the lower limit values of the numeral ranges (such as the contents of the components, the values calculated from the components, and the property values) can be appropriately combined. In the description herein, the values of the symbols in the expressions can be appropriately combined.

Therefore, in the description herein, the lower limit values and the upper limit values described in a stepwise manner for the numeral ranges of the same item can be independently combined. For example, based on the description "preferably 10 to 90, and more preferably 30 to 60" for the same item, the "preferred lower limit value (10)" and the "more preferred upper limit value (60)" can be combined to make a range of "10 to 60".

As for the numeral ranges, for example, based on the description "preferably 10 to 90, and more preferably 30 to 60", only the lower limit value can be defined as "10 or more" or "30 or more" without particularly limiting the upper limit value. Similarly, only the upper limit value can be defined as "90 or less" or "60 or less" without particularly limiting the lower limit value.

Unless otherwise indicated, the description "10 to 90" simply for the numeral range means a range of 10 or more and 90 or less.

As similar to the above, for example, based on the description "preferably 10 or more, and more preferably 30 or more" and the description "preferably 90 or less, and more preferably 60 or less" for the same item, the "preferred lower limit value (10)" and the "more preferred upper limit value (60)" can be combined to make a range of "10 or more and 60 or less". As similar to the above, only the lower limit value can be defined as " 10 or more" or "30 or more", and similarly, only the lower limit value can be defined as "90 or less" or "60 or less".

In the description herein, the expression "(meth)acrylic" is used as the concept encompassing both "methacrylic" and "acrylic". This is similarly applied to the analogous expressions including "(meth)acrylate", "(meth)acrylamide", "(meth)acryloyloxy", and the like.

In the description herein, unless otherwise indicated, the terms having the same symbol (for example, the "composition (A)" and the "monomer (a)") indicate the same item.

The method of producing an intraoral appliance equipped with a coated film according to an embodiment of the present invention is a method of producing an intraoral appliance equipped with a coated film, including coating on a prescribed surface of an intraoral appliance that is a stereolithographic article of a stereolithographic curable composition (A) containing a (meth)acrylic based polymerizable monomer (a) and a photopolymerization initiator (b), a curable composition (B) containing the same (meth)acrylic based polymerizable monomer (a) as contained in the stereolithographic curable composition (A), so as to form a coated film of a resin (B1) containing a polymer having a structure derived from the same (meth)acrylic based polymerizable monomer (a) as contained in the stereolithographic curable composition (A), on the prescribed surface of the intraoral appliance.

The intraoral appliance equipped with a coated film according to an embodiment of the present invention is an intraoral appliance equipped with a coated film, including an intraoral appliance that is a stereolithographic article of a stereolithographic curable composition (A) containing a (meth)acrylic based polymerizable monomer (a) and a photopolymerization initiator (b), and a coated film of a resin (B1) containing a polymer having a structure derived from the same (meth)acrylic based polymerizable monomer (a) as contained in the stereolithographic curable composition (A), formed on a prescribed surface of the intraoral appliance.

The method of producing an intraoral appliance equipped with a coated film described above can provide an intraoral appliance equipped with a coated film including an intraoral appliance that is a stereolithographic article of a stereolithographic curable composition (A) having formed on a prescribed surface thereof a coated film capable of imparting excellent adhesion durability and toughness. The intraoral appliance equipped with a coated film described above can be an intraoral appliance equipped with a coated film including an intraoral appliance that is a stereolithographic article of a stereolithographic curable composition (A) having formed on a prescribed surface thereof a coated film capable of imparting excellent adhesion durability and toughness.

As the mechanism providing the intraoral appliance equipped with a coated film having the aforementioned characteristics by the method of producing an intraoral appliance equipped with a coated film, the following mechanism can be exemplified while not limited thereto. Specifically, the curable composition (B) containing the same (meth)acrylic based polymerizable monomer (a) as contained in the stereolithographic curable composition (A) is coated on the intraoral appliance that is a stereolithographic article of the stereolithographic resin composition (A), and therefore it can be estimated that the penetration of the curable composition (B) to the intraoral appliance, and the interaction of the same component contained in the stereolithographic curable composition (A) and the curable composition (B), for example, hydrogen bonding, hydrophobic interaction, and π-electron interaction, develop excellent adhesion durability, and also unexpectedly enhance the toughness of the intraoral appliance itself.

In the description herein, the "coated film" means a film that is in a state capable of exhibiting the necessary function by drying or curing depending on necessity a film obtained by coating a curable composition.

Embodiments of the method of producing an intraoral appliance equipped with a coated film and the intraoral appliance equipped with a coated film will be described in detail below.

### [Intraoral Appliance equipped with Coated Film]

The intraoral appliance equipped with a coated film according to an embodiment of the present invention includes an intraoral appliance that is a stereolithographic article of a stereolithographic curable composition (A) containing a (meth)acrylic based polymerizable monomer (a) and a photopolymerization initiator (b), and a coated film of a resin (B 1) containing a polymer having a structure derived from the same (meth)acrylic based polymerizable monomer (a) as contained in the stereolithographic curable composition (A), formed on a prescribed surface of the intraoral appliance.

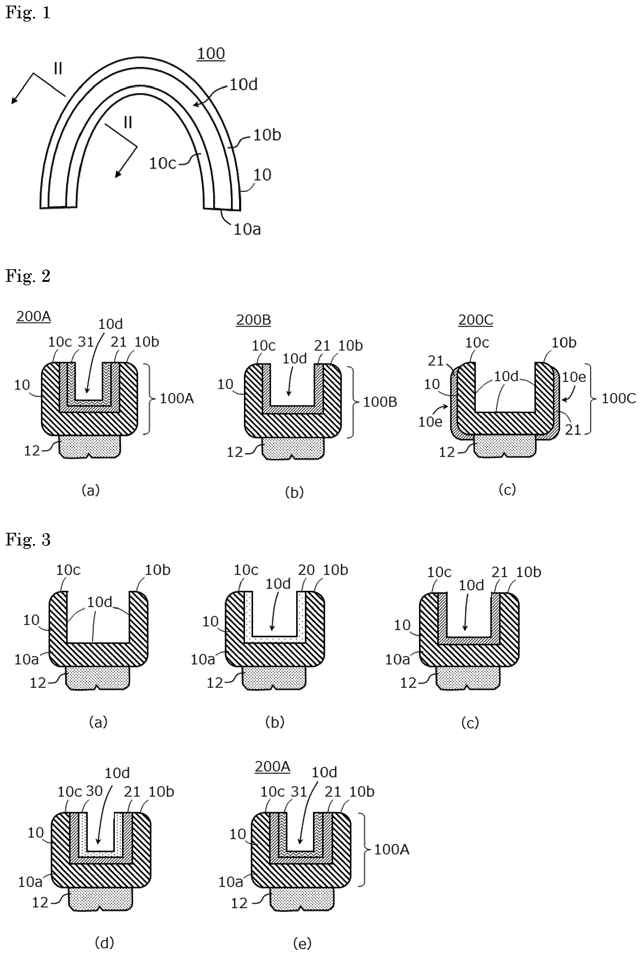

Fig. 1 is a plan view showing one example of the intraoral appliance equipped with a coated film according to an embodiment of the present invention. The intraoral appliance equipped with a coated film shown in Fig. 1 is a denture base 100 equipped with a coated film including a denture base 10 as an intraoral appliance. A prosthetic tooth may be attached the denture base 100 equipped with a coated film to provide a denture, and the denture may be designated as the intraoral appliance.

As shown in Fig. 1, the denture base 100 equipped with a coated film includes a base part 10a curbed along the alveolar ridge of the user wearing the denture base 100 equipped with a coated film, and one pair of side parts 10b and 10c to be brought into contact with the side surface of the alveolar ridge of the user, and has a concave cross sectional shape in the direction perpendicular to the extending direction of the denture base. The surfaces of the base part 10a and the side parts 10b and 10c facing the alveolar ridge of the user of the denture base constitute an alveolar ridge-contact surface 10d.

While the denture base 100 equipped with a coated film shown in Fig. 1 is for a complete denture, a partial denture may be applied, a denture for an upper jaw or a denture for a lower jaw may also be applied.

A concave part, which is not shown in the figure, for attaching a prosthetic tooth is provided around the top of the surface of the denture base 10 opposite to the alveolar ridge-contact surface 10d (i.e., the surface on the convex side), and a prosthetic tooth is attached to the concave part to constitute a denture with the denture base 10.

Fig. 2 is a cross sectional view along the line shown by the symbol II-II in Fig. 1, and is a schematic cross sectional view of the denture base equipped with a coated film as the intraoral appliance equipped with a coated film. Fig. 2 shows a denture including the denture base equipped with a coated film having a prosthetic tooth attached thereto.

A denture 200A shown in Fig. 2(a) has a denture base 100A equipped with a coated film. The denture base 100A equipped with a coated film has a coated film 21 of a resin (B1) containing a polymer having a structure derived from the same (meth)acrylic based polymerizable monomer (a) as contained in the stereolithographic curable composition (A), provided on at least a part of the alveolar ridge-contact surface 10d, and also has a backing material layer 31 provided on the coated film 21.

In the denture base 100A equipped with a coated film, the coated film 21 is provided as an adhesive layer for adhering and fixing the backing material layer 31 to the alveolar ridge-contact surface 10d.

A denture 200B shown in Fig. 2(b) has a denture base 100B equipped with a coated film. The denture base 100B equipped with a coated film has a coated film 21 of a resin (B1) containing a polymer having a structure derived from the same (meth)acrylic based polymerizable monomer (a) as contained in the stereolithographic curable composition (A), provided on at least a part of the alveolar ridge-contact surface 10d.

In the denture base 100B equipped with a coated film, the coated film 21 is provided as a backing material layer.

A denture 200C shown in Fig. 2(c) has a denture base 100C equipped with a coated film. The denture base 100C equipped with a coated film has a coated film 21 of a resin (B1) containing a polymer having a structure derived from the same (meth)acrylic based polymerizable monomer (a) as contained in the stereolithographic curable composition (A), provided on at least a part of the surface opposite to the alveolar ridge-contact surface 10d, i.e., the surface 10e on the convex side.

The denture base 10 may have an area having deteriorated lubricity, a thinned area, a scratched area, and the like on the whole or a part of the surface 10e on the convex side, due to contamination or wear during use. In the denture base 100C equipped with a coated film, the coated film 21 is provided as a coating material for covering or filling these areas.

The denture base may have both the configurations shown in Figs. 2(a) and 2(c), and may also have both the configurations shown in Figs. 2(b) and 2(c).

### <Intraoral Appliance>

The intraoral appliance is a stereolithographic article of a stereolithographic curable composition (A) containing a (meth)acrylic based polymerizable monomer (a) and a photopolymerization initiator (b).

The intraoral appliance may also be a dental occlusal splint and a mouthpiece-shaped sleep disorder treatment equipment, in addition to the denture base shown in Figs. 1 and 2. The denture base, the dental occlusal splint, or the mouthpiece-shaped sleep disorder treatment equipment is produced with a stereolithographic article of a stereolithographic curable composition (A), and thereby an intraoral appliance having a shape adapted for the user can be easily produced with less procedures. The details of the stereolithographic curable composition (A) will be described later.

### <Coated Film>

The coated film is provided on a prescribed surface of the intraoral appliance. In the case of a denture base, for example, the coated film may be provided on at least a part of the alveolar ridge-contact surface 10d to be brought into contact with the alveolar ridge of the user wearing the denture, i.e., the surface on the concave side, as shown in Figs. 2(a) and 2(b), or may be provided on at least a part of the surface opposite to the alveolar ridge-contact surface 10d, i.e., the surface 10e on the convex side, of the denture base 10 as shown in Fig. 2(c). In the case of a dental occlusal splint and a mouthpiece-shaped sleep disorder treatment equipment, the coated film may be similarly provided on at least the dentition-contact surface or at least a part of the surface opposite to the dentition-contact surface.

The coated film is formed of a resin (B1) containing a polymer having a structure derived from the same (meth)acrylic based polymerizable monomer (a) as contained in the stereolithographic curable composition (A).

The coated film is formed of a resin (B1) containing a polymer having a structure derived from the same (meth)acrylic based polymerizable monomer (a) as contained in the stereolithographic curable composition (A), and thereby a coated film capable of imparting excellent adhesion durability and toughness to the intraoral appliance as a stereolithographic article.

The intraoral appliance, such as the denture base, becomes unadapted to the oral mucosa of the user due to bone resorption, deformation of the mucosal surface, and the like, and therefore it is frequently necessary to perform backing for readapting thereto (which may also be referred to as rebasing or relining), or to perform coating for repairing an area having deteriorated lubricity, a thinned area, a scratched area, and the like due to contamination or wear.

The coated film is suitable for the coating and the backing of various kinds of an intraoral appliance that is a stereolithographic article, such as a denture base, a dental occlusal splint, and a mouthpiece-shaped sleep disorder treatment equipment, and may be provided on at least a part of the alveolar ridge-contact surface or the dentition-contact surface of the intraoral appliance, and thereby the coated film becomes a backing material layer or an adhesive layer for adhering the backing material. The coated film may also be provided on the surface opposite to the alveolar ridge-contact surface or the dentition-contact surface of the intraoral appliance, and thereby becomes a coating layer for enhancing the lubricity or filling the thinned area.

The coated film is capable of imparting excellent adhesion durability and toughness to the intraoral appliance that is a stereolithographic article, and therefore is suitable as the backing material layer, the adhesive layer for adhering the backing material, and the coating layer.

The coated film may further contain a filler, a softener, a stabilizer, a pigment, and the like as other substances than the resin (B 1). The content of the other substances than the resin (B 1) is preferably 40% by mass or less, and more preferably 20% by mass or less, based on the mass of the coated film, from the standpoint of sufficiently securing the adhesion durability to the intraoral appliance, and the like. The lower limit of the content of the other substances than the resin (B 1) in the coated film is not particularly limited, and the substances may not be contained (i.e., 0% by mass). In other words, the content of the other substances than the resin (B 1) in the coated film is preferably 0 to 40% by mass.

The coated film of the resin (B 1) can be formed by coating a curable composition (B) containing the same (meth)acrylic based polymerizable monomer (a) as contained in the stereolithographic curable composition (A) on the prescribed surface of the intraoral appliance, and then performing at least one of drying and curing depending on necessity. The details of the curable composition (B) and the resin (B 1) will be described later.

The configurations of the parts included in the intraoral appliance equipped with a coated film, and the materials and the like therefor will be described below. The method of producing an intraoral appliance equipped with a coated film will be described later.

### <Stereolithographic Curable Composition (A)>

The stereolithographic curable composition (A) contains a (meth)acrylic based polymerizable monomer (a) and a photopolymerization initiator (b).

### ((Meth)acrylic based Polymerizable Monomer (a))

Specific examples of the (meth)acrylic based polymerizable monomer (a) used in the stereolithographic curable composition (A) include a (meth)acrylate ester based polymerizable monomer and a (meth)acrylamide based polymerizable monomer. One kind of these compounds may be used alone, or two or more kinds thereof may be used in combination.

Examples of the (meth)acrylic based polymerizable monomer (a) include a polyfunctional (meth)acrylic based polymerizable monomer (a-1) having multiple polymerizable groups (which may be hereinafter referred simply to as a "polyfunctional monomer") and a monofunctional (meth)acrylic based polymerizable monomer (a-2) having one polymerizable group (which may be hereinafter referred simply to as a "monofunctional monomer"). The polyfunctional monomer and the monofunctional monomer are preferably used in combination from the standpoint of achieving the resulting stereolithographic curable composition (A) excellent in curability, and facilitating the formation of the coated film excellent in adhesion durability.

### (Polyfunctional (Meth)acrylic based Polymerizable Monomer (a-1))

The polyfunctional (meth)acrylic based polymerizable monomer (a-1) is used for imparting the curability to the stereolithographic composition (A), and for imparting the adhesion durability to the coated film to the intraoral appliance produced by stereolithography. From the standpoint of exhibiting the particularly high curability and facilitating the high adhesion durability, at least one kind selected form a urethanated (meth)acrylate ester compound, an aromatic compound based (meth)acrylate ester compound containing no urethane bond, and an alicyclic (meth)acrylate ester compound is preferably contained, at least one kind selected from a urethanated (meth)acrylate ester compound and an aromatic compound based (meth)acrylate ester compound containing no urethane bond is more preferred, and a urethanated (meth)acrylate ester compound is particularly preferred. In the case of the aromatic compound based (meth)acrylate ester compound containing no urethane bond among these, it is more preferred that at least one kind selected from the group consisting of a hydroxy group, a primary amino group, and a secondary amino group is contained. It is estimated that these compounds not only accelerate the curing through the dielectric effect of the urethane bond, the aromatic skeleton, the hydroxy group, the primary amino group, and the secondary amino group, but also form a chemical bond, such as a hydrogen bond and a π-electron effect. Among these, the urethanated (meth)acrylate ester compound having a urethane bond has a large effect of imparting toughness, and thereby cracks and breakage are difficult to occur at the adhesion interface between the intraoral appliance and the coated film, and also cracks and breakage are difficult to occur in the coated film. Therefore, the urethanated (meth)acrylate ester compound having a urethane bond facilitates the exhibition of an excellent flexural strength, and is particularly preferred as the polyfunctional (meth)acrylic based polymerizable monomer (a-1).

### (Urethanated (Meth)acrylate Ester Compound)

The urethanated (meth)acrylate ester compound can be easily synthesized, for example, by subjecting a compound containing an isocyanate containing an alkylene skeleton and a phenylene skeleton and a (meth)acrylate compound having a hydroxy group (-OH) to addition reaction. The weight average molecular weight thereof is preferably 1,000 or less from the standpoint of achieving a low viscosity facilitating the stereolithography and the coating, and facilitating the achievement of penetration to the intraoral appliance produced by stereolithography. In the case where the urethanated (meth)acrylate ester compound does not contain a polymer structure, there is no concept of weight average molecular weight, and therefore the molecular weight thereof is applied. In the case where the weight average molecular weight is 1,000 or less, the viscosity can be prevented from being increased, and the penetration can be prevented from being decreased. The weight average molecular weight is more preferably 750 or less, and further preferably 500 or less, from the standpoint of the adhesion durability to the intraoral appliance produced by stereolithography. The urethanated (meth)acrylate ester compound is preferably a compound that does not contain a polymer structure, such as polyester, polycarbonate, polyurethane, and polyether. The urethanated (meth)acrylate ester compound that does not contain the structure prevents the concentration of the functional group having polarity from being increased, and facilitates the prevention of the decrease of the adhesion durability in the presence of water, for example, in the oral cavity. The number of the urethane bond in the urethanated (meth)acrylate ester compound is preferably 3 or less, and more preferably 2 or less, per one molecule, from the standpoint of the adhesion durability in the presence of water. In other words, the number of the urethane bond in the urethanated (meth)acrylate ester compound is preferably 1 to 3 per one molecule, and more preferably 1 or 2 per one molecule. The weight average molecular weight referred herein means a polystyrene conversion weight average molecular weight obtained by gel permeation chromatography (GPC).

Examples of the compound having an isocyanate group include methylene diisocyanate (MDI), hexamethylene diisocyanate (HDI), isophorone diisocyanate (IPDI), trimethylhexamethylene diisocyanate (TMHMDI), tricyclodecane diisocyanate (TCDDI), adamantane diisocyanate (ADI), tolylene diisocyanate (TDI), xylylene diisocyanate (XDI), and diphenylmethane diisocyanate (DPMDI). Among these, HDI, IPDI, TMHMDI, and TCDDI are preferred, and IPDI and TMHMDI are more preferred, from the standpoint of the availability and the prevention of yellowing.

Examples of the compound having a hydroxy group include a hydroxy (meth)acrylate compound, such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis(4-(3-(meth)acryloyloxy-2-hydroxypropoxy)phenyl)propane, 1,2-bis(3-(meth)acryloyloxy-2-hydroxypropoxy)ethane, pentaerythritol tri(meth)acrylate, and dipentaerythritol tri- or tetra(meth)acrylate. One kind of these compounds may be used alone, or two or more kinds thereof may be used in combination. Among these, 2-hydroxyethyl (meth)acrylate, 2-hydroxypropyl (meth)acrylate, and 2-hydroxy-3-acryloyloxypropyl (meth)acrylate are preferred, and 2-hydroxyethyl (meth)acrylate is more preferred, from the standpoint of achieving the excellent adhesion durability to the intraoral appliance that is a stereolithographic article of the stereolithographic curable composition (A).

The addition reaction of the compound containing an isocyanate group which contains one or more kind selected from an alkylene skeleton and a phenylene skeleton and the (meth)acrylate compound having a hydroxy group can be performed according to the ordinary method with no particular limitation.

Examples of the urethanated (meth)acrylate ester compound that does not contain a polymer structure include 2,2,4-trimethylhexamethylenebis(2-carbamoyloxyethyl) dimethacrylate (trivial name: UDMA), 2,4-tolylenebis(2-carbamoyloxyethyl) dimethacrylate, bishydroxyethyl methacrylate-isophorone diurethane, 2,4-tolylenebis(2-carbamoyloxyethyl) dimethacrylate, N,N'-(2,2,4-trimethylhexamethylene)bis[2-(aminocarboxy)propan-1,3-diol] tetramethacrylate, hexamethylenebis(2-carbamoyloxy-3-phenoxypropyl) diacrylate, and 2,4-tolylenebis(2-carbamoyloxyethyl) hexaacrylate. One kind of these compounds may be used alone, or two or more kinds thereof may be used in combination. Among these, 2,2,4-trimethylhexamethylenebis(2-carbamoyloxyethyl) dimethacrylate and N,N'-(2,2,4-trimethylhexamethylene)bis[2-(aminocarboxy)propan-1,3-diol] tetramethacrylate are preferred, and 2,2,4-trimethylhexamethylenebis(2-carbamoyloxyethyl) dimethacrylate is more preferred, from the standpoint of the adhesion durability to the intraoral appliance that is a stereolithographic article of the stereolithographic curable composition (A).

In the case where stereolithographic curable composition (A) contains the urethanated (meth)acrylate ester compound, the total content of the urethanated (meth)acrylate ester compound in the stereolithographic curable composition (A) is preferably 10 to 100 parts by mass per 100 parts by mass of the (meth)acrylic based polymerizable monomer (a), and is more preferably 30 to 90 parts by mass, and further preferably 50 to 80 parts by mass, from the standpoint of achieving the excellent adhesion durability to the intraoral appliance that is a stereolithographic article of the stereolithographic curable composition (A).

### (Aromatic Compound based (Meth)acrylate Ester Compound containing No Urethane Bond)

Examples of the aromatic compound based (meth)acrylate ester compound containing no urethane bond include 2,2-bis((meth)acryloyloxyphenyl)propane, 2,2-bis(4-(3-acryloyloxy)-2-hydroxypropoxyphenyl)propane, 2,2-bis(4-(3-methacryloyloxy-2-hydroxypropoxy)phenyl)propane (trivial name: Bis-GMA), 2,2-bis(4-(2-(3-acryloyloxy-2-hydroxypropoxy)propyl)phenyl)propane, 2,2-bis(4-(2-(3-methacryloyloxy-2-hydroxypropoxy)propyl)phenyl)propane, 2,2-bis(4-(meth)acryloyloxyethoxyphenyl)propane, 2,2-bis(4-(meth)acyrloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl) pyromellitate. One kind of these compounds may be used alone, or two or more kinds thereof may be used in combination. Among these, a compound containing a hydroxy group is preferred, and specifically 2,2-bis(4-(3-acryloyloxy)-2-hydroxypropoxyphenyl)propane, 2,2-bis(4-(3-methacryloyloxy-2-hydroxypropoxy)phenyl)propane, 2,2-bis(4-(2-(3-acryloyloxy-2-hydroxypropoxy)propyl)phenyl)propane, 2,2-bis(4-(2-(3-methacryloyloxy-2-hydroxypropoxy)propyl)phenyl)propane are preferred, from the standpoint of achieving the excellent adhesion durability to the intraoral appliance that is a stereolithographic article of the stereolithographic curable composition (A). Among these, 2,2-bis(4-(3-methacryloyloxy-2-hydroxypropoxy)phenyl)propane is more preferred.

In the case where the stereolithographic curable composition (A) contains the aromatic compound based (meth)acrylate ester compound containing no urethane bond, the total content of the aromatic compound based (meth)acrylate ester compound containing no urethane bond in the stereolithographic curable composition (A) is preferably 5 to 90 parts by mass per 100 parts by mass of the (meth)acrylic based polymerizable monomer (a), and is more preferably 10 to 80 parts by mass, and further preferably 20 to 70 parts by mass, from the standpoint of achieving the excellent adhesion durability to the intraoral appliance produced by stereolithography.

### (Alicyclic (Meth)acrylate Ester Compound)

Examples of the alicyclic (meth)acrylate ester compound include tricyclodecanedimethanol di(meth)acrylate, cyclohexanediol di(meth)acrylate, and adamantanediol di(meth)acrylate. Among these, tricyclodecanedimethanol di(meth)acrylate is preferred.

In the case where the stereolithographic curable composition (A) contains the alicyclic (meth)acrylate ester compound, which is the polyfunctional monomer (a-1), the content of the alicyclic (meth)acrylate ester compound in the stereolithographic curable composition (A) is preferably 30 to 100% by mass based on 100% by mass of the stereolithographic curable composition (A), and is more preferably 50 to 98% by mass, and further preferably 70 to 95% by mass, from the standpoint of achieving the excellent strength of the denture.

### (Additional Polyfunctional (Meth)acrylate Ester based Polymerizable Monomer)

Examples of a polyfunctional (meth)acrylate ester based polymerizable monomer (which may be hereinafter referred to as an additional polyfunctional (meth)acrylate ester based polymerizable monomer) in the stereolithographic curable composition (A) other than the urethanated (meth)acrylate ester compound, the aromatic compound based (meth)acrylate ester compound containing no urethane bond, and the alicyclic (meth)acrylate ester compound include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, and dipentaerythritol penta(meth)acrylate. Among these, glycerol di(meth)acrylate and 1,2-bis(3-methacryloyloxy-2-hydroxypropoxy)ethane are preferred from the standpoint of achieving the excellent adhesion durability to the intraoral appliance produced by stereolithography. One kind of these compounds may be used alone, or two or more kinds thereof may be used in combination.

In the case where the stereolithographic curable composition (A) contains the additional polyfunctional (meth)acrylate ester based polymerizable monomer, the content of the additional polyfunctional (meth)acrylate ester based polymerizable monomer in the stereolithographic curable composition (A) is preferably 1 to 50% by mass, more preferably 5 to 40% by mass, and further preferably 8 to 35% by mass, based on 100% by mass of the stereolithographic curable composition (A).

In the case where the stereolithographic curable composition (A) contains the additional polyfunctional (meth)acrylate ester based polymerizable monomer, the content of the additional polyfunctional (meth)acrylate ester based polymerizable monomer in the stereolithographic curable composition (A) is preferably 1 to 50 parts by mass, more preferably 5 to 40 parts by mass, and further preferably 10 to 30 parts by mass, per 100 parts by mass as the total mass of the (meth)acrylic based polymerizable monomer (a).

The total content of the polyfunctional (meth)acrylate ester based polymerizable monomer (a-1) in the stereolithographic curable composition (A) is preferably 10 to 100 parts by mass, per 100 parts by mass as the total mass of the (meth)acrylic based polymerizable monomer (a), and is more preferably 30 to 90 parts by mass, and further preferably 50 to 80 parts by mass, from the standpoint of achieving the excellent adhesion durability to the intraoral appliance produced by stereolithography.

### (Monofunctional (meth)acrylate ester based polymerizable monomer (a-2))

The stereolithographic curable composition (A) may contain a monofunctional (meth)acrylate ester based polymerizable monomer (a-2). The monofunctional (meth)acrylate ester based polymerizable monomer (a-2) lowers the viscosity of the stereolithographic curable composition (A) and lowers the crosslinking density of the stereolithographic article, resulting in the enhancement of the penetration of the curable composition (B), and thereby the adhesion durability can be enhanced. The one polymerizable group that the monofunctional (meth)acrylate ester based polymerizable monomer (a-2) has means a (meth)acryloyl group.

Examples of the monofunctional (meth)acrylate ester based polymerizable monomer (a-2) include a (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, a linear (meth)acrylate ester based polymerizable monomer, a cyclic (meth)acrylamide compound, and a linear (meth)acrylamide compound. From the standpoint of preventing an offensive odor in handling, a (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, and a cyclic (meth)acrylamide compound, which tend to have a higher boiling point, preventing an odor, are preferred, and among these, a (meth)acrylate ester based polymerizable monomer containing an aromatic ring is more preferred from the standpoint of the curability and the adhesion durability.

A (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, and a cyclic (meth)acrylamide compound tend to have a higher boiling point and to prevent an odor as compared to a linear (meth)acrylate ester based polymerizable monomer, assuming the equivalent molecular weights therefor, and the tendency is estimated to derive from the π-electron interaction and the effect of imparting minute polarity through the fixed steric conformation. It is preferred that a urethane bond, a hydroxy group, an amino group, and an acidic group are not contained from the standpoint of the adhesion durability in water.

Examples of the (meth)acrylate ester based polymerizable monomer containing an aromatic ring include o-phenylphenol (meth)acrylate, m-phenylphenol (meth)acrylate, p-phenylphenol (meth)acrylate, methoxylated o-phenylphenol (meth)acrylate, methoxylated m-phenylphenol (meth)acrylate, methoxylated p-phenylphenol (meth)acrylate, ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, propoxylated p-phenylphenol (meth)acrylate, butoxylated o-phenylphenol (meth)acrylate, butoxylated m-phenylphenol (meth)acrylate, butoxylated p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, phenyl (meth)acrylate, 4-biphenylyl (meth)acrylate, 1-naphthyl (meth)acrylate, 2-naphthyl (meth)acrylate, anthryl (meth)acrylate, o-2-propenylphenyl (meth)acrylate, benzhydrol (meth)acrylate, cumylphenol (meth)acrylate, fluorenyl (meth)acrylate, and fluorenylmethyl (meth)acrylate.

Examples of the alicyclic (meth)acrylate ester based polymerizable monomer include 2-(1-adamantyl)propyl (meth)acrylate, 2-methyladamantyl-2-yl (meth)acrylate, 2-ethyladamantyl-2-yl (meth)acrylate, 2-n-propyladamantyl-2-yl (meth)acrylate, 2-isopropyladamantyl-2-yl (meth)acrylate, 1-(adamantan-1-yl)-1-methylethyl (meth)acrylate, 1-(adamantan-1-yl)-1-ethylethyl (meth)acrylate, 1-(adamantan-1-yl)-1-methylpropyl (meth)acrylate, and 1-(adamantan-1-yl)-1-ethylpropyl (meth)acrylate.

Examples of the cyclic (meth)acrylate ester based polymerizable monomer containing a nitrogen atom include pentamethylpiperidinyl (meth)acrylate, tetramethylpiperidinyl (meth)acrylate, and 4-(pyrimidin-2-yl)piperazin-1-yl (meth)acrylate.

Examples of the linear (meth)acrylate ester based polymerizable monomer include 2-ethylhexyl (meth)acrylate, decyl (meth)acrylate, undecyl (meth)acrylate, lauryl (meth)acrylate, tridecyl (meth)acrylate, tetradecyl (meth)acrylate, pentadecyl (meth)acrylate, cetyl (meth)acrylate, palmitoleyl (meth)acrylate, heptadecyl (meth)acrylate, oleyl (meth)acrylate, stearyl (meth)acrylate, isostearyl (meth)acrylate, 4-hydroxybutyl (meth)acrylate, 6-hydroxyhexyl (meth)acrylate, 10-hydroxydecyl (meth)acrylate, glycerol mono(meth)acrylate, erythritol mono(meth)acrylate, and isobornylcyclohexyl (meth)acrylate.

Examples of the cyclic (meth)acrylamide compound include N-(meth)acryloylmorpholine, N-(meth)acryloylpyrrolidine, N-(meth)acryloylpiperidine, N-(meth)acryloyl-2-methylpiperidine, and N-(meth)acryloyl-2,2,6,6-tetramethylpiperidine.

Examples of the linear (meth)acrylamide compound include N,N-dimethyl(meth)acrylamide, N,N-diethyl(meth)acrylamide, N,N-di-n-propyl(meth)acrylamide, N-isopropyl(meth)acrylamide, N,N-di-n-butyl(meth)acrylamide, N,N-di-n-hexyl(meth)acrylamide, N,N-di-n-octyl(meth)acrylamide, N,N-di-2-ethylhexyl(meth)acrylamide, N-(2-hydroxyethyl)(meth)acrylamide, and N,N,N-bis(2-hydroxyethyl)(meth)acrylamide.

Among these, the monofunctional (meth)acrylate ester based polymerizable monomer (a-2) is preferably o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, o-phenylphenol (meth)acrylate, m-phenylphenol (meth)acrylate, p-phenylphenol (meth)acrylate, methoxylated o-phenylphenol (meth)acrylate, methoxylated m-phenylphenol (meth)acrylate, methoxylated p-phenylphenol (meth)acrylate, ethoxylated o-phenylphenol (meth)acrylate, ethoxylated m-phenylphenol (meth)acrylate, ethoxylated p-phenylphenol (meth)acrylate, fluorenyl (meth)acrylate, fluorenylmethyl (meth)acrylate, and 4-biphenylyl (meth)acrylate, more preferably m-phenoxybenzyl (meth)acrylate, o-phenoxybenzyl (meth)acrylate, p-phenoxybenzyl (meth)acrylate, o-phenylphenol (meth)acrylate, m-phenylphenol (meth)acrylate, p-phenylphenol (meth)acrylate, ethoxylated o-phenylphenol (meth)acrylate, ethoxylated m-phenylphenol (meth)acrylate, and ethoxylated p-phenylphenol (meth)acrylate, further preferably m-phenoxybenzyl (meth)acrylate and ethoxylated o-phenylphenol (meth)acrylate, and still further preferably m-phenoxybenzyl acrylate and ethoxylated o-phenylphenol acrylate, from the standpoint of achieving the high curability of the stereolithographic curable composition (A) and the excellent adhesion durability of the curable composition (B) to the intraoral appliance that is a stereolithographic article of the stereolithographic curable composition (A).

The total content of the monofunctional (meth)acrylate ester based polymerizable monomer (a-2) in the stereolithographic curable composition (A) is preferably 1 to 60 parts by mass per 100 parts by mass as the total mass of the (meth)acrylic based polymerizable monomer (a), and is more preferably 5 to 50 parts by mass, and further preferably 10 to 40 parts by mass, from the standpoint of achievement of the further excellent adhesion durability to the intraoral appliance that is a stereolithographic article of the stereolithographic curable composition (A).

The (meth)acrylic based polymerizable monomer (a) contained in stereolithographic curable composition (A) may be constituted substantially only by the polyfunctional (meth)acrylate ester based polymerizable monomer (a-1), or the polyfunctional (meth)acrylate ester based polymerizable monomer (a-1) and the monofunctional (meth)acrylate ester based polymerizable monomer (a-2). The fact that the (meth)acrylic based polymerizable monomer (a) is constituted substantially only by the polyfunctional (meth)acrylate ester based polymerizable monomer (a-1), or the polyfunctional (meth)acrylate ester based polymerizable monomer (a-1) and the monofunctional (meth)acrylate ester based polymerizable monomer (a-2) means the total content of the additional (meth)acrylic based polymerizable monomer (a) other than the polyfunctional (meth)acrylate ester based polymerizable monomer (a-1), or the polyfunctional (meth)acrylate ester based polymerizable monomer (a-1) and the monofunctional (meth)acrylate ester based polymerizable monomer (a-2) is less than 10.0 parts by mass, preferably less than 5.0 parts by mass, more preferably less than 1.0 part by mass, further preferably less than 0.1 part by mass, and particularly preferably less than 0.01 part by mass, per 100 parts by mass as the total mass of the (meth)acrylic based polymerizable monomer (a) contained in the stereolithographic curable composition (A).

### (Photopolymerization Initiator (b))

The photopolymerization initiator (b) used in the stereolithographic curable composition (A) may be selected from polymerization initiators that have been used in the ordinary industries, and among these, a photopolymerization initiator used in the dental field is preferred.

Examples of the photopolymerization initiator (b) include a (bis)acylphosphine oxide compound, a thioxanthone compound or a quaternary ammonium salt of a thioxanthone compound, a ketal compound, an α-diketone compound, a coumarin compound, an anthraquinone compound, a benzoin alkyl ether compound, and an α-aminoketone based compound. One kind of these photopolymerization initiator (b) may be used alone, or two or more kinds thereof may be used in combination.

Among these photopolymerization initiators (b), at least one kind selected from the group consisting of a (bis)acylphosphine oxide compound and an α-diketone compound is preferably used. With the use thereof, the stereolithographic curable composition (A) that is excellent in photocurability in the ultraviolet region and the visible light region, and shows the sufficient photocurability by using any of light sources including a laser, a halogen lamp, a light emitting diode (LED), and a xenon lamp.

In the (bis)acylphosphine oxide compound, examples of the acylphosphine oxide compound include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyldi-(2,6-dimethylphenyl) phosphonate, 2,4,6-trimethylbenzoylphenylphosphine oxide sodium salt, 2,4,6-trimethylbenzoylphenylphosphine oxide potassium salt, and 2,4,6-trimethylbenzoyldiphenylphosphine oxide ammonium salt. Examples of the bisacylphosphine oxide compound include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide. Examples thereof also include the compounds described in JP2000-159621A.

Among the (bis)acylphosphine oxide compounds, at least one of 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and 2,4,6-trimethylbenzoylphenylphosphine oxide sodium salt is particularly preferably used as the photopolymerization initiator (b).

Examples of the α-diketone compound include diacetyl, benzyl, camphorquinone, 2,3-pentadione, 2,3-octadione, 9,10-phenanthrenequinone, 4,4'-oxybenzyl, and acenaphthenequinone. Among these, camphorquinone is particularly preferred in the case where a light source in the visible light region is used.

The total content of the photopolymerization initiator (b) in the stereolithographic curable composition (A) is not particularly limited, and the content of the photopolymerization initiator (b) is preferably 0.01 to 20 parts by mass per 100 parts by mass as the total mass of the (meth)acrylic based polymerizable monomer (a) from the standpoint of the curability and the like of the resulting stereolithographic curable composition (A). In the case where the content of the photopolymerization initiator (b) is 0.01 part by mass or more, the polymerization sufficiently proceeds, facilitating the formation of the molded article. The content of the photopolymerization initiator (b) is more preferably 0.05 part by mass or more, more preferably 0.1 part by mass or more, and particularly preferably 0.5 part by mass or more, per 100 parts by mass as the total mass of the (meth)acrylic based polymerizable monomer (a). In the case where the content of the photopolymerization initiator (b) is 20 parts by mass or less, the polymerization initiator can be prevented from being deposited from the stereolithographic curable composition (A) even through the solubility thereof is low. The content of the photopolymerization initiator (b) is more preferably 15 parts by mass or less, further preferably 10 parts by mass or less, and particularly preferably 5 parts by mass or less, per 100 parts by mass as the total mass of the (meth)acrylic based polymerizable monomer (a).

The stereolithographic curable composition (A) is not particularly limited, as far as containing the (meth)acrylic based polymerizable monomer (a) and the photopolymerization initiator (b), and for example, may further contain other components than these. The stereolithographic curable composition (A) can be produced according to the known method.

The stereolithographic curable composition (A) may contain a polymerization accelerator in a range that does not impair the effects of the present invention, for the purpose of enhancing the photocuring capability. Examples of the polymerization accelerator include ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-N,N-dimethylaminobenzoate, 4-(N,N-dimethylamino)benzophenone, and butyl 4-(N,N-dimethylamino)benzoate. Among these, at least one selected from the group consisting of ethyl 4-(N,N-dimethylamino)benzoate, n-butoxyethyl 4-(N,N-dimethylamino)benzoate, and 4-(N,N-dimethylamino)benzophenone is preferably used from the standpoint of imparting the excellent curability to the stereolithographic curable composition (A).

The stereolithographic curable composition (A) may contain a filler for regulating the liquid properties. Examples of the filler include an organic filler, an inorganic filler, and an organic-inorganic composite filler. One kind of the filler may be used alone, or two or more kinds thereof may be used in combination.

Examples of the material of the organic filler include polymethyl methacrylate, polyethyl methacrylate, a methyl methacrylate-ethyl methacrylate copolymer, crosslinked polymethyl methacrylate, crosslinked polyethyl methacrylate, polyester, polyamide, polycarbonate, polyphenylene ether, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. One kind of the materials may be used alone, or two or more kinds thereof may be used in combination. The shape of the organic filler is not particularly limited, and the particle diameter of the filler can be appropriately selected in use.

Examples of the material of the inorganic filler include quartz, silica, alumina, silica-titania, silica-titania-barium oxide, silica-zirconia, silica-alumina, lanthanum glass, borosilicate glass, soda glass, barium glass, strontium glass, glass ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. One kind of the materials may be used alone, or two or more kinds thereof may be used in combination. The shape of the organic filler is not particularly limited, and an irregular shape filler or a spherical filler can be appropriately selected in use.

Examples of the organic-inorganic composite filler include an organic-inorganic composite filler obtained by dispersing an inorganic based filler in an organic based filler, and an organic-inorganic composite filler obtained by coating an inorganic based filler with various kinds of a polymerizable monomer.

The content of the filler in the stereolithographic curable composition (A) may be 0 part by mass or more per 100 parts by mass of the stereolithographic curable composition (A), and in the case where the stereolithographic curable composition (A) contains the filler, for example, the content of the filler is preferably 0.1 part by mass or more, more preferably 0.3 part by mass or more, and further preferably 0.5 part by mass or more, per 100 parts by mass of the stereolithographic curable composition (A), from the standpoint of regulating the viscosity of the composition, imparting the strength to the denture base, and the like. The content of the filler in the stereolithographic curable composition (A) is preferably 20 parts by mass or less, more preferably 10 parts by mass or less, and further preferably 5 parts by mass or less, per 100 parts by mass of the stereolithographic curable composition (A), from the standpoint of preventing the influence on the stereolithographic capability due to the increase of the viscosity of the stereolithographic curable composition (A) and the standpoint of achieving the excellent strength and toughness of the denture. In other words, the content of the filler is preferably 0 to 20 parts by mass, and more preferably 0.1 to 20 parts by mass, per 100 parts by mass of the stereolithographic curable composition (A).

The stereolithographic curable composition (A) may contain a polymer for the purpose of modifying the flexibility, the flowability, and the like, in such a range that does not impair the spirit of the present invention. For example, natural rubber, synthetic isoprene rubber, liquid isoprene rubber or a hydrogenated product thereof, polybutadiene rubber, liquid polybutadiene rubber or a hydrogenated product thereof, styrene-butadiene rubber, chloroprene rubber, ethylene-propylene rubber, acrylic rubber, isoprene-isobutylene rubber, acrylonitrile-butadiene rubber, or a styrene based elastomer may be added. Specific examples of other polymers that can be added include a polystyrene-polyisoprene-polystyrene block copolymer, a polystyrene-polybutadiene-polystyrene block copolymer, a poly(α-methylstyrene)-polybutadiene-poly(α-methylstyrene) block copolymer, a poly(p-methylstyrene)-polybutadiene-poly(p-methylstyrene) block copolymer, and hydrogenated products thereof.

The stereolithographic curable composition (A) may contain a softener depending on necessity. Examples of the softener include a petroleum based softener, such as paraffin based, naphthene based, and aromatic based process oils, and a vegetable oil based softener, such as paraffin, peanut oil, and rosin. One kind of the softeners may be used alone, or two or more kinds thereof may be used in combination. The total content of the softener is not particularly limited unless the spirit of the present invention is impaired, and is generally 200 parts by mass or less, and preferably 100 parts by mass or less, per 100 parts by mass as the total mass of the (meth)acrylic based polymerizable monomer (a).

The stereolithographic curable composition (A) may contain a known stabilizer for the purpose of preventing the deterioration or regulating the photocurability. Examples of the stabilizer include a polymerization inhibitor, an ultraviolet ray absorbent, and an antioxidant.

Examples of the polymerization inhibitor include hydroquinone, hydroquinone monomethyl ether, dibutylhydroquinone, dibutylhydroquinone monomethyl ether, 4-t-butylcatechol, 2-t-butyl-4,6-dimethylphenol, 2,6-di-t-butylphenol, and 3,5-di-t-butyl-4-hydroxytoluene. The content of the polymerization inhibitor is preferably 0.001 to 5.0 parts by mass per 100 parts by mass as the total mass of the (meth)acrylic based polymerizable monomer (a).

The stereolithographic curable composition (A) may contain a known additive for the purpose of regulating the color tone or regulating the paste properties. Examples of the additive include a pigment, a dye, a thickener, a polymer, a prepolymer (oligomer), and an aryl compound (such as an aryl iodonium salt). One kind of the additives may be used alone, or two or more kinds thereof may be used in combination.

### <Curable Composition (B)>

The curable composition (B) contains the same (meth)acrylic based polymerizable monomer (a) as contained in the stereolithographic curable composition (A) containing the (meth)acrylic based polymerizable monomer (a) and the photopolymerization initiator (b).

In the case where stereolithographic curable composition (A) contains multiple kinds of a (meth)acrylic based polymerizable monomers (a) as the (meth)acrylic based polymerizable monomer (a), it is preferred that the polymerizable monomer that has the largest content therein is contained in the curable composition (B) as the common polymerizable monomer.

In the (meth)acrylic based polymerizable monomer (a) contained in the curable composition (B), the content of the same (meth)acrylic based polymerizable monomer (a) as contained in the stereolithographic curable composition (A) is preferably 20% by mass or more, more preferably 30% by mass or more, further preferably 40% by mass or more, still further preferably 50% by mass or more, still more further preferably 55% by mass or more, even further preferably 60% by mass or more, and even still further preferably 65% by mass or more, and is, for example, 100% by mass or less, preferably 98% by mass or less, more preferably 95% by mass or less, further preferably 90% by mass or less, still further preferably 85% by mass or less, and still more further preferably 80% by mass or less, from the standpoint of enhancing the adhesion durability and the toughness. In other words, in the (meth)acrylic based polymerizable monomer (a) contained in the curable composition (B), the content of the same (meth)acrylic based polymerizable monomer (a) as contained in the stereolithographic curable composition (A) is preferably 20 to 100% by mass.

The content of the (meth)acrylic based polymerizable monomer (a) in the curable composition (B) is preferably 10% by mass or more, more preferably 20% by mass or more, and further preferably 50% by mass or more, and may be 100% by mass with no particular problem, from the standpoint of enhancing the excellent adhesion durability to the intraoral appliance that is a stereolithographic article. In other words, the content of the (meth)acrylic based polymerizable monomer (a) in the curable composition (B) is preferably 10 to 100% by mass.

The curable composition (B) preferably contains the polyfunctional (meth)acrylic based polymerizable monomer (a-1) as the (meth)acrylic based polymerizable monomer (a) from the standpoint of facilitating the securement of the adhesion durability and the toughness of the coated film. In particular, both the stereolithographic curable composition (A) and the curable composition (B) preferably contain the polyfunctional (meth)acrylic based polymerizable monomer (a-1) as the (meth)acrylic based polymerizable monomer (a) from the standpoint of further facilitating the securement of the adhesion durability and the toughness of the coated film.

In this case,
(i) it is more preferred that both the stereolithographic curable composition (A) and the curable composition (B) contain the urethanated (meth)acrylate ester compound or the aromatic compound based (meth)acrylate ester compound containing no urethane bond as the polyfunctional (meth)acrylic based polymerizable monomer (a-1), and
(ii) it is further preferred that both the stereolithographic curable composition (A) and the curable composition (B) contain the urethanated (meth)acrylate ester compound as the polyfunctional (meth)acrylic based polymerizable monomer (a-1).

The curable composition (B) preferably contains the monofunctional (meth)acrylic based polymerizable monomer (a-2) as the (meth)acrylic based polymerizable monomer (a), in addition to the polyfunctional (meth)acrylic based polymerizable monomer (a-1) from the standpoint of facilitating the enhancement of the adhesion durability.

In this case,
(iii) it is more preferred that both the stereolithographic curable composition (A) and the curable composition (B) contain the urethanated (meth)acrylate ester compound as the polyfunctional (meth)acrylic based polymerizable monomer (a-1), and the curable composition (B) contains the monofunctional (meth)acrylic based polymerizable monomer (a-2), and
(iv) it is further preferred that both the stereolithographic curable composition (A) and the curable composition (B) contain the urethanated (meth)acrylate ester compound as the polyfunctional (meth)acrylic based polymerizable monomer (a-1), and the curable composition (B) contains the (meth)acrylate ester based polymerizable monomer containing an aromatic ring as the monofunctional (meth)acrylic based polymerizable monomer (a-2).

In particular, both the stereolithographic curable composition (A) and the curable composition (B) preferably contains the polyfunctional (meth)acrylic based polymerizable monomer (a-1) and the monofunctional (meth)acrylic based polymerizable monomer (a-2) as the (meth)acrylic based polymerizable monomer (a) from the standpoint of further facilitating the enhancement of the adhesion durability.

In this case,
(v) it is more preferred that both the stereolithographic curable composition (A) and the curable composition (B) contain the urethanated (meth)acrylate ester compound as the polyfunctional (meth)acrylic based polymerizable monomer (a-1), and both the stereolithographic curable composition (A) and the curable composition (B) contain the monofunctional (meth)acrylic based polymerizable monomer (a-2), and
(vi) it is further preferred that both the stereolithographic curable composition (A) and the curable composition (B) contain the urethanated (meth)acrylate ester compound as the polyfunctional (meth)acrylic based polymerizable monomer (a-1), and both the stereolithographic curable composition (A) and the curable composition (B) contain the (meth)acrylate ester based polymerizable monomer containing an aromatic ring as the monofunctional (meth)acrylic based polymerizable monomer (a-2).

The curable composition (B) may contain a polymerizable monomer that is not contained in the stereolithographic curable composition (A), an organic solvent, and an additive, in such a range that does not impair the spirit of the present invention, for regulating the thickness of the coated film, and regulating the penetration to the stereolithographic article.

Examples of the organic solvent include ethyl acetate, butyl acetate, methylene chloride, acetone, ethanol, isopropanol, and methyl methacrylate. One kind of the organic solvents may be used alone, or two or more kinds thereof may be used in combination. Among these, ethyl acetate, ethanol, and isopropanol are preferred from the standpoint of the safety and the appropriate volatility. Examples of the additive include a polymerization accelerator, a filler, a polymer, a softener, a stabilizer, and a pigment, as similar to the stereolithographic curable composition (A) described above.

### <Resin (B 1)>

The resin (B 1) constituting the coated film provided on the prescribed surface of the intraoral appliance contains a polymer having a structure derived from the same (meth)acrylic based polymerizable monomer (a) as contained in the stereolithographic curable composition (A).

The resin (B 1) preferably contains the polymer having a structure derived from the same (meth)acrylic based polymerizable monomer (a) as contained in the stereolithographic curable composition (A) in an amount of 50% by mass or more, more preferably 70% by mass or more, further preferably 85% by mass or more, and still further preferably 90% by mass or more, from the standpoint of facilitating the securement of the adhesion durability and the toughness of the coated film. The upper limit thereof is not particularly limited, may be 100% by mass, may be 99% by mass or less, and may be 98% by mass or less. In other words, the content of the polymer having a structure derived from the same (meth)acrylic based polymerizable monomer (a) as contained in the stereolithographic curable composition (A) in the resin (B 1) is preferably 50 to 100% by mass.

The coated film of the resin (B 1) can be formed by coating the curable composition (B) containing the same (meth)acrylic based polymerizable monomer (a) as contained in the stereolithographic curable composition (A) on the prescribed surface of the intraoral appliance, and then performing at least one of drying and curing depending on necessity. The curable composition (B) has been described above, and the details of the formation method of the resin (B 1) will be described later.

### [Method of producing Intraoral Appliance equipped with Coated Film]

The method of producing an intraoral appliance equipped with a coated film according to an embodiment of the present invention is a method of producing an intraoral appliance equipped with a coated film, including coating on a prescribed surface of an intraoral appliance that is a stereolithographic article of a stereolithographic curable composition (A) containing a (meth)acrylic based polymerizable monomer (a) and a photopolymerization initiator (b), a curable composition (B) containing the same (meth)acrylic based polymerizable monomer (a) as contained in the stereolithographic curable composition (A), so as to form a coated film of a resin (B 1) containing a polymer having a structure derived from the same (meth)acrylic based polymerizable monomer (a) as contained in the stereolithographic curable composition (A), on the prescribed surface of the intraoral appliance.

The prescribed surface of the intraoral appliance, on which the curable composition (B) is coated, is not particularly limited, and in the case where backing is performed, is preferably the whole or a part of the alveolar ridge-contact surface or the dentition-contact surface of the intraoral appliance. In the case where the coating is performed, the prescribed surface is preferably the whole or a part of the surface opposite to the alveolar ridge-contact surface or the dentition-contact surface of the intraoral appliance.

The method of producing an intraoral appliance equipped with a coated film will be described below with reference to specific examples and the drawings.

### <Production Method for Backing>

Fig. 3 is a schematic process chart showing one example of the method of producing an intraoral appliance equipped with a coated film according to the present embodiment, and specifically shows one example of the method of producing an intraoral appliance equipped with a coated film by performing backing on an alveolar ridge-contact surface of a denture base. Fig. 3 shows an example of the production process in the case where a denture 200A including the denture base 100A shown in Fig. 2(a).

### (Step 1A)

A denture base 10 having a base part 10a and one pair of side parts 10b and 10c as shown in Fig. 3(a) is produced in advance through stereolithography of the stereolithographic curable composition (A) containing the (meth)acrylic based polymerizable monomer (a) and the photopolymerization initiator (b).

The stereolithography herein can be performed by various known methods.

The stereolithographic article may be subjected to secondary polymerization after stereolithography, for polymerizing the unreacted polymerizable monomer, by such a method as light irradiation with a dental light irradiator, a secondary polymerization device for stereolithography, or the like. The secondary polymerization performed can allow the polymerization of the stereolithographic article to proceed highly. The polymerization can also be allowed to proceed by storing the stereolithographic article for a long period of time.

The following step 2A may be performed before the secondary polymerization or the polymerization by long-term storage of the stereolithographic article.

A prosthetic tooth 12 is attached to the resulting denture base 10 at a concave part, which is not shown in the figure, for attaching a prosthetic tooth, so as to constitute a denture.

### (Step 2A)

Subsequently, before starting the use of the denture by a user wearing the denture, or after using the denture by a user wearing the denture for a certain period of time, the curable composition (B) containing the same (meth)acrylic based polymerizable monomer (a) as contained in the stereolithographic curable composition (A) is coated on at least the necessary portion of the alveolar ridge-contact surface 10d of the denture base 100, so as to form an uncured curable composition layer 20 as shown in Fig. 3(b). The coating method of the curable composition (B) may be various known methods, examples of which include a dipping method and a spraying method. The curable composition (B) may be manually coated with a brush or the like.

The uncured curable composition layer 20 is then dried depending on necessity and cured to form a coated film 21 of the resin (B 1) containing a polymer having a structure derived from the same (meth)acrylic based polymerizable monomer (a) as contained in the stereolithographic curable composition (A) on the alveolar ridge-contact surface 10d of the denture base 100 as shown in Fig. 3(c).

It is possible that after performing the secondary polymerization of the stereolithographic article or allowing the polymerization to proceed highly by storing the stereolithographic article for a long period of time in the step 1A described above, the stereolithographic article obtained by performing the secondary polymerization of the stereolithographic article or allowing the polymerization to proceed highly after the stereolithography is used as the intraoral appliance, and the curable composition (B) is coated on the prescribed surface of the stereolithographic article to form the coated film of the resin (B 1) on the prescribed surface in the step 2A.

According to the procedure, the hardness and the strength of the stereolithographic article can be enhanced, but it can be estimated that there may be a disadvantage from the standpoint of the adhesiveness between the coated film of the resin (B 1) and the stereolithographic article. However, as described above, the coated film is formed by using the curable composition (B) containing the same (meth)acrylic based polymerizable monomer (a) as contained in the stereolithographic curable composition (A), and thereby the good adhesion durability can be secured even though the coated film is formed after the secondary polymerization of the stereolithographic article. Furthermore, there is also an advantage that it is not necessary to form the coated film before the polymerization proceeds highly, resulting in a large degree of freedom in timing of providing the coated film.

In alternative, after subjecting the stereolithographic curable composition (A) to stereolithography in the step 1A as described above, the curable composition (B) may be coated on the prescribed surface of the stereolithographic article before the secondary polymerization or the stereolithographic article before allowing the polymerization to proceed highly in the step 2 above, so as to form the coated film of the resin (B1) on the prescribed surface. According to the procedure, the adhesion durability between the stereolithographic article and the coated film can be further easily enhanced.

In the case where the coated film is formed on the stereolithographic article before the secondary polymerization, the secondary polymerization of the stereolithographic article may be performed simultaneously with the drying and curing for forming the coated film, or the secondary polymerization of the stereolithographic article may be performed before the drying and curing for forming the coated film or after the drying and curing for forming the coated film.

In the case where the coated film itself is used as the backing material layer, the following step 3A is not necessary.

### (Step 3A)

Subsequently, a backing material composition is coated to form an uncured backing material layer 30 as shown in Fig. 3(d), and then the uncured backing material layer 30 is dried depending on necessity and cured by allowing to stand for a certain period of time or by retaining to a prescribed temperature for a certain period of time, so as to form a backing material layer 31 as shown in Fig. 3(e). Thereafter, machining, such as grinding and polishing, is performed depending on necessity. Consequently, a denture base equipped with a coated film or a denture equipped with a coated film is obtained as an intraoral appliance equipped with a coated film.

The backing material composition used in the step 3Amay be a commercially available backing material for a denture base or the various known backing materials.

### <Production Method with Coating>

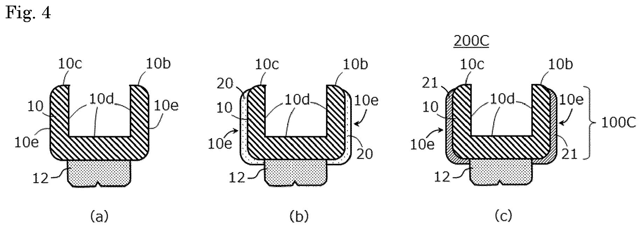

Fig. 4 is a schematic process chart showing another example of the method of producing an intraoral appliance equipped with a coated film, and specifically shows one example of the method of producing an intraoral appliance equipped with a coated film by performing coating on a surface opposite to an alveolar ridge-contact surface of a denture base. Fig. 4 shows an example providing a denture 200C including the denture base 100C shown in Fig. 2(c).

### (Step 1B)

A denture base 10 that is a stereolithographic article is produced in the same procedure as in the step 1A described with reference to Fig. 3, to which a prosthetic tooth 12 is attached to provide a denture as shown in Fig. 4(a). In the step 1B, the stereolithographic article may be subjected to secondary polymerization, or the polymerization thereof may be allowed to proceed highly by storing for a long period of time, or in alternative, the following step 2B may be performed without performing the secondary polymerization and the highly proceeding polymerization of the stereolithographic article in the step 1B.

### (Step 2B)

The curable composition (B) containing the same (meth)acrylic based polymerizable monomer (a) as contained in the stereolithographic curable composition (A) is coated on at least the necessary portion of the surface 10e on the convex side of the denture base 10, so as to form an uncured curable composition layer 20 as shown in Fig. 4(b). The uncured curable composition layer 20 is then dried depending on necessity and cured to form a coated film 21 of the resin (B 1) containing a polymer having a structure derived from the same (meth)acrylic based polymerizable monomer (a) as contained in the stereolithographic curable composition (A) on the surface 10e on the convex side of the denture base 10 (Fig. 4(c)).

It is possible that after performing the secondary polymerization of the stereolithographic article or allowing the polymerization to proceed highly in the step 1B described above, the curable composition (B) is coated on the surface on the convex side of the stereolithographic article to form the coated film of the resin (B 1) in the step B2. It is also possible that the step 2B is performed without performing the secondary polymerization and the highly proceeding polymerization of the stereolithographic article in the step 1B, and the secondary polymerization of the stereolithographic article is performed after coating the curable composition (B) on the surface on the convex side of the stereolithographic article.

After the step 2B, machining, such as grinding and polishing, is performed depending on necessity. The machining performed forms cutting marks, polishing marks, or the like on the surface of the coated film, and as a result, the coated film has machining marks. Consequently, a denture base equipped with a coated film or a denture equipped with a coated film is obtained as an intraoral appliance equipped with a coated film.

### <Curing of Curable Composition>

In applying the coating or the backing to the intraoral appliance that is a stereolithographic article of the stereolithographic curable composition (A), or in other words, in the step (2A) or (2B), it is preferred that the curable composition (B) is coated on the target surface of the coating or the target surface of the backing of the stereolithographic article to provide a curable composition layer, which is dried depending on necessity, and then cured through light irradiation.

The light irradiation for curing the curable composition layer may be performed by using any of a known dental light irradiator (α-Light V, available from J. Morita Tokyo Mfg. Corp.) and a post-cure apparatus (Otoflash G171, available from Envision TEC, Inc.). In particular, the light energy used for curing the stereolithographic curable composition (A) is preferably an active energy ray. "The active energy ray" means an energy ray that is capable of curing the stereolithographic curable composition (A), such as an ultraviolet ray, an electron beam, an X-ray, a radiation, and a high frequency wave.

Examples of the source of the active energy ray include an illumination, such as a halogen lamp, a xenon lamp, a metal halide lamp, an LED, a mercury lamp, and a fluorescent lamp, and a halogen lamp and an LED are particularly preferred.

While the post-cure apparatus for stereolithography allows to select an air stream or a nitrogen stream in some cases, a nitrogen stream is preferably selected for the purpose of forming only coating from the standpoint that the surface is favorably cured to achieve smoothness, and an air stream is preferably selected for the purpose of forming an adhesive coated film for backing.

### Examples

The present invention will be described more specifically with reference to examples below, but the present invention is not limited to the examples.

The components used in the stereolithographic curable compositions (A) in Examples and Comparative Examples and abbreviated names thereof will be described below.

### [(Meth)acrylate based Polymerizable Monomer (a)]

### <Polyfunctional (Meth)acrylate based Polymerizable Monomer (a-1)>

- UDMA: 2,2,4-trimethylhexamethylenebis(2-carbamoyloxyethyl) dimethacrylate (available from Kyoeisha Chemical Co., Ltd., molecular weight: 471)
- Bis-GMA: 2,2-bis(4-(3-methacryloyloxy)-2-hydroxypropoxyphenyl)propane (available from Shin-Nakamura Chemical Co., Ltd.)
- TCDDMA: tricyclodecanedimethanol dimethacrylate (available from Kyoeisha Chemical Co., Ltd.)
- TEGDMA: triethylene glycol dimethacrylate (available from Shin-Nakamura Chemical Co., Ltd.)

### <Monofunctional (Meth)acrylate based Polymerizable Monomer (a-2)>

- POBA: m-phenoxybenzyl acrylate (available from Kyoeisha Chemical Co., Ltd.)
- EPPA: ethoxylated o-phenylphenol acrylate (available from Shin-Nakamura Chemical Co., Ltd.)

### [Photopolymerization Initiator (b)]

- TPO: 2,4,6-trimethylbenzoyldiphenylphosphine oxide

### [Polymerization Inhibitor]

- BHT: 3,5-di-t-butyl-4-hydroxytoluene

### <Reference Example 1>

### [Production of Stereolithographic Curable Composition (A)-1]

700 parts by mass of UDMA, 300 parts by mass of POBA, 30 parts by mass of TPO, and 5.0 parts by mass of BHT were placed in a 2 L brown polyethylene wide mouthed bottle, to which a mechanical agitator was inserted, and the components were agitated at 25°C for 48 hours and ensured complete dissolution. The resulting composition was designated as the stereolithographic curable composition (A)-1.

### <Reference Example 2>

### [Production of Stereolithographic Curable Composition (A)-2]

600 parts by mass of Bis-GMA, 400 parts by mass of POBA, 30 parts by mass of TPO, and 5.0 parts by mass of BHT were placed in a 2 L brown polyethylene wide mouthed bottle, to which a mechanical agitator was inserted, and the components were agitated at 25°C for 48 hours and ensured complete dissolution. The resulting composition was designated as the stereolithographic curable composition (A)-2.

### <Reference Example 3>

### [Production of Stereolithographic Curable Composition (A)-3]

900 parts by mass of TCDDMA, 100 parts by mass of EPPA, 30 parts by mass of TPO, and 5.0 parts by mass of BHT were placed in a 2 L brown polyethylene wide mouthed bottle, to which a mechanical agitator was inserted, and the components were agitated at 25°C for 48 hours and ensured complete dissolution. The resulting composition was designated as the stereolithographic resin curable composition (A)-3.

### <Reference Example 4>

### [Production of Stereolithographic Curable Composition (A)-4]

700 parts by mass of UDMA, 300 parts by mass of TEGDMA, 30 parts by mass of TPO, and 5.0 parts by mass of BHT were placed in a 2 L brown polyethylene wide mouthed bottle, to which a mechanical agitator was inserted, and the components were agitated at 25°C for 48 hours and ensured complete dissolution. The resulting composition was designated as the stereolithographic curable composition (A)-4.

### <Reference Example 5>

### [Production of Stereolithographic Curable Composition (A)-5]

900 parts by mass of TCDDMA, 100 parts by mass of TEGDMA, 30 parts by mass of TPO, and 5.0 parts by mass of BHT were placed in a 2 L brown polyethylene wide mouthed bottle, to which a mechanical agitator was inserted, and the components were agitated at 25°C for 48 hours and ensured complete dissolution. The resulting composition was designated as the stereolithographic curable composition (A)-5.

The compositions of the stereolithographic curable composition (A)-1 to (A)-5 are shown in Table 1.

**Table 1**

| | | Name of component | (A)-1 | (A)-2 | (A)-3 | (A)-4 | (A)-5 |
|---|---|---|---|---|---|---|---|
| Mixing amount (part by mass) | Polyfunctional monomer (a-1) | UDMA | 700 | - | - | 700 | - |
| | | Bis-GMA | - | 600 | - | - | - |
| | | TCDDMA | - | - | 900 | - | 900 |
| | | TEGDMA | - | - | - | 300 | 100 |
| | Monofunctional monomer (a-2) | POBA | 300 | 400 | - | - | - |
| | | EPPA | - | - | 100 | - | - |
| | Photopolymerization initiator (b) | TPO | 30 | 30 | 30 | 30 | 30 |
| | Polymerization inhibitor | BHT | 5 | 5 | 5 | 5 | 5 |

### [Examples 1 to 7 and Comparative Examples 1 to 5]

The compositions (A)-1 to (A)-5 prepared in Reference Examples 1 to 5 each were used as the stereolithographic curable composition (A) as shown in Table 2, and subjected to stereolithography to produce a stereolithographic article. The compositions (A)-1 to (A)-5 prepared in Reference Examples 1 to 5 and a composition prepared by mixing the composition (A)-1 prepared in Reference Example 1 and ethyl acetate (available from Fujifilm Wako Pure Chemical Corporation) at the mass ratio shown in Table 2 each were used as the curable composition (B). The curable composition (B) was coated on the stereolithographic article to form a coated film, thereby producing a test piece including the stereolithographic article having a coated film of a resin formed thereon. The resulting test piece was evaluated for the adhesion durability and the toughness. Specifically, the test piece was produced, and measured and evaluated for the characteristics, according to the following procedures.

### <Adhesion Durability of Coated Film>

The stereolithographic curable compositions (A)-1, (A)-2, (A)-3, (A)-4, and (A)-5 obtained in Reference Examples 1 to 5 each were subjected to stereolithography with a stereolithography machine (Digitalwax (registered trademark) 020D, available from DWS S.r.l.), so as to produce an article having a diameter of 20 mm and a height of 15 mm, which was flashed 2,000 times with a light irradiator (Otoflash (registered trademark) G171, available from Envision TEC, Inc.) to complete curing. After completing curing, one plane surface was polished with silicon carbide paper #600 (available from Nihon Kenshi Co., Ltd.) under running water, and then water on the surface was blown off with a dental air syringe, so as to provide a stereolithographic article.

The curable composition (B) was coated with a brush on the polished surface of the resulting stereolithographic article according to the combination of the composition (A) and the composition (B) shown in Tables 2 and 3, and after thinly spreading with a dental syringe, was subjected to flash irradiation 1,000 times with a light irradiator (Otoflash (registered trademark) G171, available from Envision TEC, Inc.) under a nitrogen stream. A silicone sheet having a thickness of approximately 1.0 mm having a circular hole having a diameter of 4 mm was adhered to the coated surface of the curable composition (B), and the adhesion area was determined. Subsequently, a backing material for a denture base (Kuraribase (registered trademark), available from Kuraray Noritake Dental Inc.) was charged in the circular hole without coating, onto which a PET film of 1 cm × 1 cm was pressed, and then a weight of 500 g was placed on the assembly via a glass plate of 1 cm square with a thickness of 5.0 mm, followed by allowing to stand at room temperature for 1 hour, so as to cure the backing material for a denture base. Thereafter, the weight and the PET film were removed to produce a specimen for an adhesion durability test. 10 test specimens were produced, in which five specimens were stored in water at 37°C for one day, taken out from water, wiped for draining off water, the stored in a thermostat chamber retained to 37°C for 24 hours, and then measured for the shearing adhesion strength by using a universal testing machine (Autograph (registered trademark) AG-I 100kN, available from Shimadzu Corporation). The shearing adhesion strength was measured at a crosshead speed set to 1 mm/min. The average value of the measured values for the five test pieces was designated as the shearing adhesion strength. The remaining five test specimens were subjected to a thermal cycle load of immersing in a water bath at 4°C and a water bath at 60°C each for 1 minute alternately 4,000 times, then taken out from water, wiped for draining off water, and then measured for the shearing adhesion strength. The adhesion durability was evaluated by the shearing adhesion strength after the thermal cycle load.

In this test, an adhesion strength of 5 MPa or more is a clinically usable adhesion strength, that of 10 MPa or more is a good adhesion strength equivalent to the ordinary products, and that of 15 MPa or more is a significantly excellent adhesion strength exceeding the ordinary products.

### <Toughness>

The stereolithographic curable compositions (A)-1 to (A)-5 obtained in Reference Examples each were used to prepare a rectangular solid having a dimension of a length of 64.0 mm, a width of 10.0 mm, and a thickness of 3.3 mm described in JIS T6501:2012 (Acrylic Denture Base Resins) through stereolithography by using a stereolithography machine (Digitalwax (registered trademark) 020D, available from DWS S.r.l.) by using a stereolithography machine (Digitalwax (registered trademark) 020D, available from DWS S.r.l.). The stereolithographic article was then flashed 2,000 times with a light irradiator (Otoflash (registered trademark) G171, available from Envision TEC, Inc.) to complete curing. Subsequently, the curable composition (B) was coated with brush on the resulting stereolithographic article according to the combination of the curable composition (A) and the curable composition (B) of Examples and Comparative Examples except for Comparative Example 6, and after thinly spreading with a dental syringe, was subjected to flash irradiation 1,000 times with a light irradiator (Otoflash (registered trademark) G171, available from Envision TEC, Inc.). Thus, a toughness test piece having a dimension of a length of 64.0 mm, a width of 10.0 mm, and a thickness of 3.3 mm described in JIS T6501:2012 (Acrylic Denture Base Resins) was obtained.

In Comparative Example 6, a test piece was produced in the same manner including the flash irradiation as above except that the step of coating the curable composition (B) with a brush was not performed.

The toughness test pieces of Examples and Comparative Examples each were stored in water at 37°C for one day and then evaluated by subjecting to a toughness test. Specifically, a toughness test (n = 5) was performed by using a universal testing machine (Autograph AG-I 100kN, available from Shimadzu Corporation) at a crosshead speed of 5 mm/min.

The average values of the measured values are shown in Tables 2 and 3. The toughness of the test piece is preferably 100 MPa or more, and more preferably 120 MPa or more, in terms of flexural strength. The displacement at break thereof is preferably no breakage.

As for the displacement at break, the case where breakage occurred at a displacement of 10 mm or more was evaluated as good (A), the case where breakage occurred at a displacement of more than 5 mm and less than 10 mm was evaluated as moderate (B), and the case where breakage occurred at a displacement of 5 mm or less was evaluated as poor (C), in which the grade B or better was evaluated acceptable.

The measurement results of the properties in Examples and Comparative Examples are shown in Tables 2 and 3 with the compositions of the stereolithographic curable compositions (A) and the curable compositions (B).

**Table 2**

| | | | Example | | | | | | | |
|---|---|---|---|---|---|---|---|---|---|---|
| | | | 1 | 2 | 3 | 4 | 5 | 6 | 7 | 8 |
| Stereolithographic curable composition (A) | | | (A)-1 | (A)-2 | (A)-3 | (A)-4 | (A)-5 | (A)-1 | (A)-1 | (A)-4 |
| Curable composition (B) | Component (part by mass) | Curable composition (A)-1 | 100 | - | - | - | - | 30 | 15 | 100 |
| | | Curable composition (A)-2 | - | 100 | - | - | - | - | - | - |
| | | Curable composition (A)-3 | - | - | 100 | - | - | - | - | - |
| | | Curable composition (A)-4 | - | - | - | 100 | - | - | - | - |
| | | Curable composition (A)-5 | - | - | - | - | 100 | - | - | - |
| | | Ethyl acetate | - | - | - | - | - | 70 | 85 | - |
| Shearing adhesion strength after storing in water at 37°C for 24 hours (MPa) | | | 16.8 | 16.3 | 12.8 | 11.6 | 9.5 | 12.8 | 9.8 | 13.1 |
| Shearing adhesion strength after thermal cycle 4,000 times (MPa) | | | 16.4 | 15.8 | 11.5 | 10.9 | 9.1 | 12.2 | 9.7 | 12.2 |
| Toughness | | Flexural strength (MPa) | 132 | 124 | 113 | 111 | 102 | 121 | 107 | 115 |
| | | Displacement at break | A | A | B | B | B | A | B | B |

**Table 3**

| | | | Comparative Example | | | | | |
|---|---|---|---|---|---|---|---|---|
| | | | 1 | 2 | 3 | 4 | 5 | 6 |
| Stereolithogra phic curable composition (A) | | | (A)-1 | (A)-2 | (A)-3 | (A)-4 | (A)-5 | (A)-3 |
| Curable composition (B) | Component (part by mass) | Curable composition (A)-1 | - | 100 | - | - | 100 | - |
| | | Curable composition (A)-2 | - | - | 100 | - | - | - |
| | | Curable composition (A)-3 | - | - | - | 100 | - | - |
| | | Curable composition (A)-4 | - | - | - | - | - | - |
| | | Curable composition (A)-5 | 100 | - | - | - | - | - |
| | | Ethyl acetate | - | - | - | - | - | - |
| Shearing adhesion strength after storing in water at 37°C for 24 hours (MPa) | | | 6.4 | 8.8 | 5.8 | 4.1 | 3.8 | - |
| Shearing adhesion streng h after thermal cycle 4,000 times (MPa) | | | 2.9 | 4.2 | 2.3 | 2.1 | 2.3 | - |
| Toughness | | Flexural strength (MPa) | 98 | 96 | 85 | 82 | 77 | 97 |
| | | Displacement at break | C | C | C | C | C | C |

As shown in Tables 2 and 3, in Examples 1 to 8, the coated film of the resin (B1) containing a polymer having a structure derived from the same (meth)acrylic based polymerizable monomer (a) as contained in the stereolithographic curable composition (A) is formed on the stereolithographic article of the stereolithographic curable composition (A). It is understood that according to the configuration, the coated film is excellent in adhesion durability and toughness.

It is understood from the comparison between Example 1 and Example 3 and the comparison between Example 4 and Example 5 that the coated film is further excellent in adhesion durability and toughness in the case where the curable composition (A)-1 or (A)-4 containing UDMA, which is a urethanated (meth)acrylate ester compound containing a urethane bond, is used as the stereolithographic curable composition (A) and curable composition (B). It is also apparent from Example 8 that the coated film is excellent in adhesion durability and toughness as compared to Comparative Examples in the case where the curable composition (A)-1 containing only UDMA, which is a urethanated (meth)acrylate ester compound containing a urethane bond, as the polyfunctional monomer (a-1) is used in one of the stereolithographic curable composition (A) and the curable composition (B), and the curable composition (A)-4 containing UDMA and TEGDMA, which is the additional polyfunctional (meth)acrylate ester based polymerizable monomer, as the polyfunctional monomer (a-1) is used in the other one thereof.

In Comparative Examples 1 to 5, on the other hand, the coated film of the resin containing a polymer having a structure derived from the (meth)acrylic based polymerizable monomer (a) that is different from that is contained in the stereolithographic curable composition (A) is formed on the stereolithographic article of the stereolithographic curable composition (A). It is understood that as a result, the stereolithographic articles equipped with a coated film of Comparative Examples 1 to 5 are inferior in adhesion durability and toughness to the stereolithographic articles equipped with a coated film of Examples 1 to 8. It is also understood that the stereolithographic article of Comparative Example 6 having no coated film is inferior in toughness.

### Reference Sign List

10: Denture base
10a: Base part
10b, 10c: Side part
10d: Alveolar ridge-contact surface (surface on concave side)
10e: Surface opposite to alveolar ridge-contact surface (surface on convex side)
12: Prosthetic tooth
20: Uncured curable composition layer
21: Coated film
30: Uncured backing material layer
31: Backing material layer
100, 100A, 100B, 100C: Denture base equipped with coated film
200A, 200B, 200C: Denture

## Claims

1. A method of producing an intraoral appliance equipped with a coated film, comprising coating on a prescribed surface of an intraoral appliance that is a stereolithographic article of a stereolithographic curable composition (A) containing a (meth)acrylic based polymerizable monomer (a) and a photopolymerization initiator (b), a curable composition (B) containing the same (meth)acrylic based polymerizable monomer (a) as contained in the stereolithographic curable composition (A), so as to form a coated film of a resin (B 1) containing a polymer having a structure derived from the same (meth)acrylic based polymerizable monomer (a) as contained in the stereolithographic curable composition (A), on the prescribed surface of the intraoral appliance.

2. The method of producing an intraoral appliance equipped with a coated film according to claim 1, wherein the curable composition (B) contains the same (meth)acrylic based polymerizable monomer (a) as contained in the stereolithographic curable composition (A) in a mass proportion in effective ingredients of 10% by mass or more.

3. The method of producing an intraoral appliance equipped with a coated film according to claim 1 or 2, wherein the method comprises coating the curable composition (B) on an alveolar ridge-contact surface, which is a surface to be brought into contact with an alveolar ridge, of the intraoral appliance, so as to form a coated film of the resin (B 1) on the alveolar ridge-contact surface of the intraoral appliance.

4. The method of producing an intraoral appliance equipped with a coated film according to any one of claims 1 to 3, wherein the stereolithographic curable composition (A) and the curable composition (B) contain a polyfunctional (meth)acrylic based polymerizable monomer (a-1) as the (meth)acrylic based polymerizable monomer (a).

5. The method of producing an intraoral appliance equipped with a coated film according to claim 4, wherein the polyfunctional (meth)acrylic based polymerizable monomer (a-1) contains a urethanated (meth)acrylic compound.

6. The method of producing an intraoral appliance equipped with a coated film according to claim 5, wherein the stereolithographic curable composition (A) and the curable composition (B) further contain a monofunctional (meth)acrylic based polymerizable monomer (a-2) as the (meth)acrylic based polymerizable monomer (a).

7. The method of producing an intraoral appliance equipped with a coated film according to claim 6, wherein the monofunctional (meth)acrylic based polymerizable monomer (a-2) contains a (meth)acrylate ester based polymerizable monomer containing an aromatic ring.

8. The method of producing an intraoral appliance equipped with a coated film according to any one of claims 1 to 7, wherein the intraoral appliance has been subjected to secondary polymerization after stereolithography, and the method comprises coating the curable composition (B) on a prescribed surface of the stereolithographic article having been subjected to secondary polymerization, so as to form a coated film of the resin (B 1) on the prescribed surface.

9. The method of producing an intraoral appliance equipped with a coated film according to any one of claims 1 to 7, wherein the method comprises, after stereolithography of the stereolithographic curable composition (A), coating the curable composition (B) on a prescribed surface of the stereolithographic article before secondary polymerization, so as to form a coated film of the resin (B1) on the prescribed surface.

10. The method of producing an intraoral appliance equipped with a coated film according to any one of claims 1 to 9, wherein the method comprises machining the coated film after forming the coated film.

11. An intraoral appliance equipped with a coated film, comprising
an intraoral appliance that is a stereolithographic article of a stereolithographic curable composition (A) containing a (meth)acrylic based polymerizable monomer (a) and a photopolymerization initiator (b), and
a coated film of a resin (B 1) containing a polymer having a structure derived from the same (meth)acrylic based polymerizable monomer (a) as contained in the stereolithographic curable composition (A), formed on a prescribed surface of the intraoral appliance.

12. The intraoral appliance equipped with a coated film according to claim 11, wherein the resin (B 1) contains the polymer having a structure derived from the same (meth)acrylic based polymerizable monomer (a) as contained in the stereolithographic curable composition (A) in an amount of 50% by mass or more.

13. The intraoral appliance equipped with a coated film according to claim 11 or 12, wherein the intraoral appliance has the coated film of the resin (B 1) on an alveolar ridge-contact surface, which is a surface to be brought into contact with an alveolar ridge, of the intraoral appliance.

14. The intraoral appliance equipped with a coated film according to any one of claims 11 to 13, wherein the intraoral appliance is a denture base.

15. The intraoral appliance equipped with a coated film according to any one of claims 11 to 14, wherein the coated film has machining marks.
